# EUROPEAN PATENT SPECIFICATION

(11) **EP 1 572 298 B1**
(45) Date of publication and mention of the grant of the patent: **07.04.2010**
(21) Application number: 03775527.9
(22) Date of filing: 13.11.2003
(51) Int. Cl.: A61N 5/10, A61B 6/03

(54) **RADIOTHERAPY APPARATUS**
STRAHLENTHERAPIE-GERÄT
APPAREIL DE RADIOTHERAPIE

(30) Priority: 28.11.2002 GB 0227729
(43) Date of publication of application: 14.09.2005
(73) Proprietor: Elekta AB (publ), 103 93 Stockholm (SE)
(72) Inventor: Brown, Kevin John, Horsham, West Sussex RH13 5EJ (GB)
(74) Representative: Downing, Michael Philip
(86) International application number: PCT/GB2003/004881
(87) International publication number: WO 2004/047923

(56) References cited:
- EP-A- 0 562 585
- WO-A-01/60236
- WO-A-98/02091
- US-A- 5 748 700
- US-A1- 2003 048 868

## Description

### FIELD OF THE INVENTION

The present invention relates to a radiotherapy apparatus.

### BACKGROUND ART

Existing radiotherapy apparatus seeks to direct a beam of radiation to a tumour within a patient in order to cause the death of cancerous cells. By its nature, the radiation is harmful and efforts are therefore made to limit the application of the radiation to healthy areas of the patient. Typically, these involve the use of collimators for the beam, and directional techniques in which the beam is directed toward the tumour from a variety of directions, thereby maximising the dose at the tumour site and minimising it in adjacent areas.

Such techniques depend on the operator being aware of the position of the tumour. This knowledge will usually be gained from previous investigations and from off-line analysis carried out after the treatment for use in refining subsequent treatments. However, soft tissue is apt to move randomly even over short periods of time, and this places a limitation on the use of historic information.

This problem is particularly acute in the treatment of prostate cancer. The prostate is located close to other structures such as the rectum and bladder, both of which are sensitive to the effects of radiation at therapeutic doses. Undesirable side-effects resulting from irradiation of these parts are easily produced if care is not taken. However, movement of these structures and the prostate is common, and can be between 3 and 8mm. This distance is significantly greater than the separation between the structures and thus presents a difficulty in treatment.

### SUMMARY OF THE INVENTION

It has been proposed to combine a radiotherapeutic accelerator with a computerised tomography (CT) apparatus in order to locate the current position of the prostate and direct the radiation beam accordingly. The three-dimensional result of the CT scan would be presented to the operator to allow identification of the prostate and confirmation of the proposed volume to be irradiated. This arrangement offers much greater precision in treatment provided it can be controlled.

The document WO-A-01/60236 discloses a radiation therapy system that includes a radiation source that moves about a path and directs a beam of radiation towards an object and a cone-beam computer tomography system (CBCT). The cone-beam computer tomography system includes an x-ray source that emits an x-ray beam in a cone-beam form towards an object to be imaged and an amorphous silicon flat-panel imager receiving x-rays after they pass through the object, the imager providing an image of the object. A computer is connected to the radiation source and the cone beam computerized tomography system, wherein the computer receives the image of the object and based on the image sends a signal to the radiation source that controls the path of the radiation source.

For purposes of intercomparison between the CBCT and a conventional CT scan, six slices from the cone beam computerized tomography data set were averaged to produce a slice thickness equivalent to that of the conventional scan. The images were displayed at comparable window and level to allow comparison.

One difficulty is that three-dimensional (holographic) displays are not available and the dataset must therefore be projected onto a two dimensional screen. A trace must then be made on this two dimensional screen of the three dimensional structure to be irradiated. Suitable software can analyse the dataset and propose irradiation patterns, but the proposal must still be understandable to the operator so that it can be validated.

The present invention therefore proposes a radiotherapy apparatus as defined in claim 1.

Such sectional images are familiar in style to operators who are accustomed to working with "portal" images derived during treatment from radiation which passes through the patient. However, the sectional images will offer better contrast and will show the detail of a section rather than that of a projection. It will be easier to interpret and visualise a series of sectional views than a three dimensional view.

The sectional views are preferably arranged orthogonally for ease of visualisation. At least three sectional views are useful since each section offers two degrees of freedom, and thus three will offer six. These can be cross-checked to ensure that the dataset is reliable and that the operator has not made a gross error.

The views will ideally intersect substantially at the isocentre of the therapeutic source as the region of interest is likely to be at that point, and the best accuracy of the therapeutic accelerator is in that vicinity.

Each sectional view will be a digital image comprising a number of pixels. A simple approach would be to create pixels which correspond to a 2D plane of voxels in the tomography dataset. However, an image derived in this way is likely to contain a significant amount of noise. This is normally removed by averaging nearby pixels, but the cost of this is a reduced resolution. In a typical CT set, the resolution is about 1mm³ per voxel and thus a loss of resolution is likely to be problematic.

In the present invention, the sectional nature of the images that are provided allow pixels to be the result of averaging a plurality of voxels arranged transverse to the section, typically orthogonal and ideally in a linear relationship. In this way, the resolution of the CT set can be retained whilst reducing noise in the image by averaging. Compared to a display of the 3d dataset, this offers the prospect of an improved quality image at the same resolution, which is also easier to interpret. Typically, good results can be achieved using between 5 and 20 voxels. About 10 is suitable, ie between 7 and 15.

There is a ideally a display means for showing the sectional views to an operator. The therapeutic source can then be controlled on the basis of instructions from the operator, given via an input means such as a mouse or other pointer, which is preferably correlated to the display. This correlation can be via a superimposed image on the display which is moveable in response to operation of the input means. The superimposed image can be derived from one of a previous investigation and a treatment of the patient, so that it corresponds in shape to the area or the image which is being sought. The superimposed image is preferably an outline so that the underlying image is clear.

### BRIEF DESCRIPTION OF THE DRAWINGS

An embodiment of the present invention will now be described, by way of example, with reference to the accompanying figures, in which;
Figure 1 shows a schematic illustration of the treatment apparatus of the present invention;
Figure 2 shows the data handling apparatus;
Figure 3 is a perspective view of the dataset typically acquired from CT apparatus with the data of interest highlighted;
Figure 4 shows the sectional views obtained by the present invention; and
Figure 5 shows the conversion from a 3D dataset to a 2D image.

### DETAILED DESCRIPTION OF THE EMBODIMENT

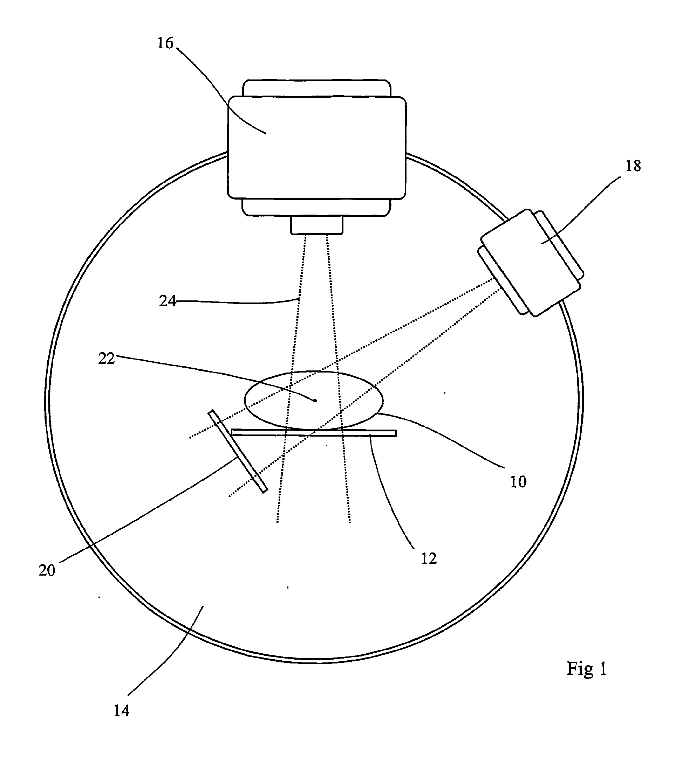

Referring to figure 1, the patient 10 is placed on a couch 12 or other support. At the foot (or head) of the couch is a rotatable stage 14 on which is mounted a treatment source 16 for generating therapeutic radiation, a diagnostic source 18 for generating imaging radiation, and a flat panel imager 20 of (for example) amorphous silicon. Such imagers are able to convert incident imaging radiation into visible images or (in this case) a digital image output by associating the imager with a CCD or photodiode array.

The stage 14 is rotatable about a horizontal axis which co-incides with the isocentre 22. The isocentre is the nominal centre of the volume to be treated and is thus a short distance above the patient table. The sources 16, 18 are aligned to direct radiation towards the isocentre. The diagnostic source 18 is offset from the treatment source 16 by a rotation about the axis of the stage to allow both a clear view toward the patient. The imager 20 is thus aligned on the stage 14 so as to be opposite the diagnostic source 18.

The rotational offset between the sources 16, 18 is sufficient to place the imager 20 outside the radiation field 24 of the treatment source 16. This protects the imager 20 which could be damaged through exposure to the MeV energy radiation of the treatment source 16 as opposed to the keV energy radiation of the diagnostic source.

A source able to generate both MeV and keV radiation could replace both treatment and diagnostic sources 16, 18. In this case, it would probably be necessary to arrange for the imager to be moveable out of the radiation field during treatment. An apparatus of this type is shown in GB-A-2353458.

Thus, as the stage 14 rotates, both sources 16, 18 and the imager 20 rotate together about the patient and stay in alignment. This allows the diagnostic source 18 and the imager 20 to create a complete cone beam CT dataset. It also allows the treatment source 16 to direct radiation toward an affected area of the patient from a number of alignments, thereby limiting the dose applied to unaffected areas around the region to be treated.

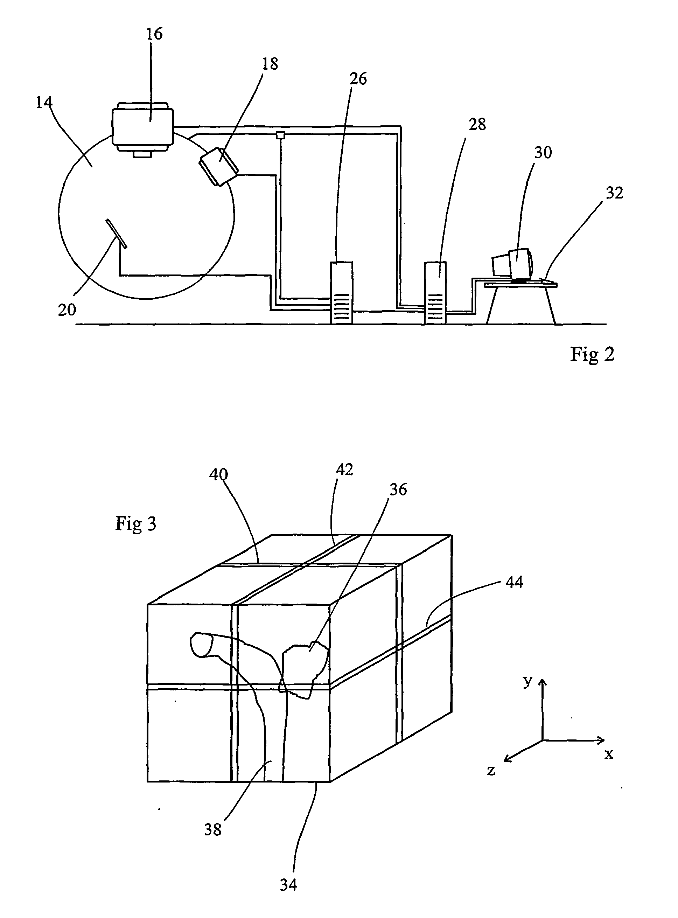

Figure 2 shows the data handling apparatus. The diagnostic source 18 and the stage 14 are controlled by a reconstruction server 26 which also collects the data from the imager 20 and reconstructs this into a three-dimensional CT dataset. This is made available to a treatment control computer 28 which has a display 30 and input means 32 for interaction with an operator. Once treatment decisions are made, these are used to control the treatment source 16 and the stage 14.

Figure 3 shows the CT dataset 34 in a 2D projection. Structures within the dataset 34 include an enlarged prostate 36 and rectum 38. In this view, it is difficult to discern the relative location in three dimensions of the different parts of the image. It is possible to analyse the dataset through software and identify structures, but the nature of software and the number of possible configurations means that verification by a operator is advisable. This then requires comprehension of the dataset by the operator.

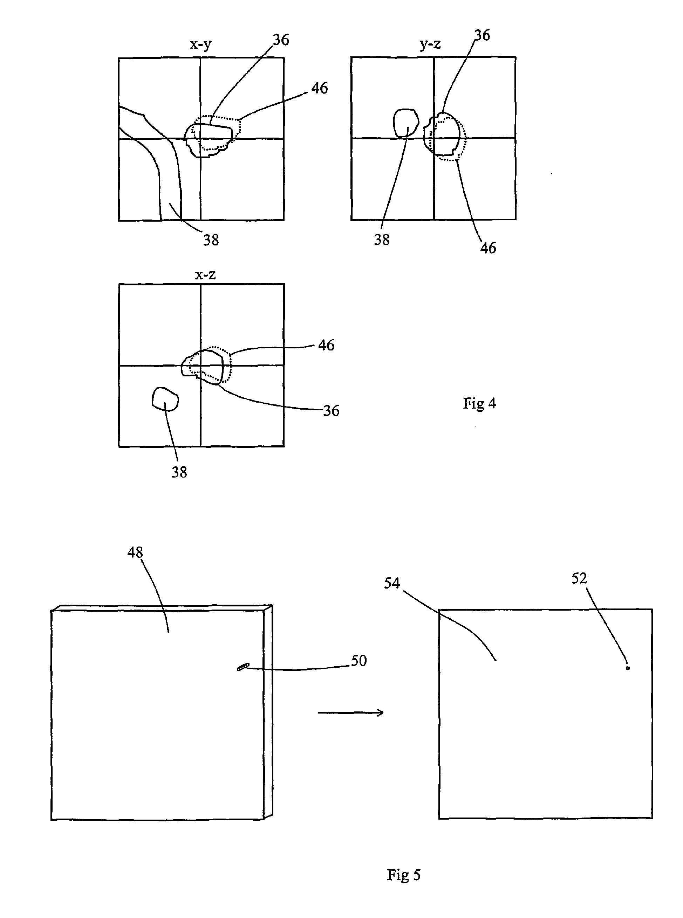

Three orthogonal slices 40, 42, 44 are therefore taken from the dataset 34. These intersect at the isocentre located in the middle of the dataset 34. As the apparatus is usually targeted at the isocentre, these slices will typically include the region of interest. The three slices are then shown on the display 30 as in figure 4. From these images, the distinction between the prostate 36 and rectum 38 (in this case) is more apparent. The apparatus superimposes an image 46 of the prostate to be treated, taken either from an earlier diagnostic investigation of the patient, or from data gathered in a previous treatment. This is shown in dotted in figure 4,although in practice it may be advisable to show the image in a contrasting colour.

It can be seen from figure 4 that the prostate has moved since collection of the image; this is normal and is due to pressure exerted by the rectum and bladder resulting from food and drink digested by the patient. The rectum and bladder also move, which means that in the absence of contemporaneous location data, the radiation field must be defined so as to avoid both the previous locations of these structures and also a safety margin around them. This means that it is unlikely that the entirety of the prostate will be irradiated, thereby compromising the treatment.

Through the use of contemporaneous CT data, the actual locations of target and non-target structures can be ascertained and the dose adjusted accordingly. The operator can manipulate the image 46 so as to overlay the prostate 36, and the control computer uses this information to direct the treatment source 16. Software of this type is known, for example iView^{™} software available from the applicant.

The slices shown by the software are similar to "portal" images, ie images taken from therapeutic radiation which has passed through the patient and been attenuated. Operators are accustomed to interpreting portal images and are therefore able to interpret the images of the present invention with ease. Portal images are not suitable for use in this way, however, as the aim is to avoid applying therapeutic levels of radiation to the patient until the proposed treatment has been validated.

Figure 5 shows how the image quality can be improved in the above arrangement. A single slice 48 is extracted from the dataset as set out above. A three-dimensional dataset comprises a 3D array of voxels, each having an associated intensity etc. The slice is between 5 and 20 voxels thick, typically 10 thick. The software selects a line of voxels 50 which extend in the thickness of the slice 48. The intensity of these voxels are then averaged and that average value is used for the intensity value of the pixel 52 in the corresponding image 54.

As a result, local transients in the image are smoothed away and the image loses much of its graininess. Structures become easier to discern. However, there is no loss of image resolution as would normally be the case when averaging.

It will be appreciated that many variations may be made to the above embodiment, without departing from the scope of the present invention.

## Claims

1. Radiotherapy apparatus adapted to provide therapeutic radiation and imaging radiation comprising a two-dimensional imager (20) for the imaging radiation, a computing means (26) adapted for processing an output of the two-dimensional imager to produce tomography data (34) including a plurality of intersecting sectional views (40, 42, 44), each sectional view being an image (54) containing pixels (52) with values derived from averaging a plurality of voxels (50) in the tomography data set which are disposed transverse to the corresponding section; and a therapeutic source (16) controllable in response to feedback from the tomography data to produce therapeutic radiation.

2. Radiotherapy apparatus according to claim 1 in which the sectional views are arranged orthogonally.

3. Radiotherapy apparatus according to claim 1 in which at least three sectional views are prepared.

4. Radiotherapy apparatus according to claim 3 in which the views intersect substantially at the isocentre of the therapeutic source.

5. Radiotherapy apparatus according to claim 1 in which the plurality of voxels are disposed orthogonal to the corresponding section.

6. Radiotherapy apparatus according to claim 5 in which the plurality of voxels are disposed linearly.

7. Radiotherapy apparatus according to any one of claims 5 to 6 in which between 5 and 20 voxels are employed.

8. Radiotherapy apparatus according to any one of claims 5 to 7 in which between 7 and 15 voxels are employed.

9. Radiotherapy apparatus according to any one of claims 5 to 8 in which about 10 voxels are employed.

10. Radiotherapy apparatus according to any one of the preceding claims In which there is a display means for showing the sectional views.

11. Radiotherapy apparatus according to claim 10 in which the therapeutic source is controlled on the basis of instructions from an operator, given via an input means.

12. Radiotherapy apparatus according to claim 11 in which the input means is correlated to the display.

13. Radiotherapy apparatus according to claim 12 in which the correlation is via a superimposed image on the display which is moveable in response to operation of the input means.

14. Radiotherapy apparatus according to claim 13 in which the superimposed image is derived from one of a previous investigation and a treatment of the patient.

15. Radiotherapy apparatus according to claim 13 or claim 14 in which the superimposed image is an outline.

## Patentansprüche

1. Radiotherapiegerät, das angepasst ist, therapeutische Strahlung und bildgebende Strahlung bereitzustellen, wobei das Gerät einen zweidimensionalen Imager (20) für die bildgebende Strahlung, ein Rechenmittel (26) umfasst, das zum Verarbeiten einer Ausgabe des zweidimensionalen Imagers angepasst ist, um Tomographiedaten (34) einschließlich einer Mehrheit sich schneidender Schnittansichten (40, 42, 44) zu produzieren, wobei jede Schnittansicht ein Bild (54) ist, das Pixel (52) mit Werten enthält, die sich von der Mittelung einer Mehrheit von Voxeln (50) im Tomographiedatensatz herleiten, die quer zum entsprechenden Teilbereich angeordnet sind; und eine therapeutische Quelle (16) umfasst, die als Reaktion auf Feedback seitens der Tomographiedaten steuerbar ist, um therapeutische Strahlung zu erzeugen.

2. Radiotherapiegerät nach Anspruch 1, wobei die Schnittansichten orthogonal angeordnet sind.

3. Radiotherapiegerät nach Anspruch 1, wobei zumindest drei Schnittansichten vorbereitet werden.

4. Radiotherapiegerät nach Anspruch 3, wobei sich die Ansichten im Wesentlichen im Isozentrum der therapeutischen Quelle schneiden.

5. Radiotherapiegerät nach Anspruch 1, wobei die Mehrheit der Voxel orthogonal zum entsprechenden Teilbereich angeordnet sind.

6. Radiotherapiegerät nach Anspruch 5, wobei die Mehrheit der Voxel linear angeordnet sind.

7. Radiotherapiegerät nach einem beliebigen der Ansprüche 5 bis 6, wobei zwischen 5 und 20 Voxel verwendet werden.

8. Radiotherapiegerät nach einem beliebigen der Ansprüche 5 bis 7, wobei zwischen 7 und 15 Voxel verwendet werden.

9. Radiotherapiegerät nach einem beliebigen der Ansprüche 5 bis 8, wobei ca.10 Voxel verwendet werden.

10. Radiotherapiegerät nach einem beliebigen der vorhergehenden Ansprüche, wobei ein Display-Mittel zum Anzeigen der Schnittansichten vorhanden ist.

11. Radiotherapiegerät nach Anspruch 10, wobei die therapeutische Quelle auf der Basis von Instruktionen einer Bedienungsperson gesteuert wird, die über ein Eingabemittel gegeben werden.

12. Radiotherapiegerät nach Anspruch 11, wobei das Eingabemittel mit dem Display korreliert ist.

13. Radiotherapiegerät nach Anspruch 12, wobei die Korrelation über ein auf dem Display eingeblendetes Bild geschieht, das als Reaktion auf die Betätigung des Eingabemittels bewegbar ist.

14. Radiotherapiegerät nach Anspruch 13, wobei sich das eingeblendete Bild von einem einer vorherigen Untersuchung und einer Behandlung des Patienten herleitet.

15. Radiotherapiegerät nach Anspruch 13 oder Anspruch 14, wobei das eingeblendete Bild eine Kontur ist.

## Revendications

1. Un appareil de radiothérapie adapté de façon à émettre un rayonnement et une imagerie thérapeutiques, comprenant un imageur bidimensionnel (20) pour le rayonnement d'imagerie, un dispositif de calcul (26) adapté pour le traitement d'une signal de sortie de l'imageur bidimensionnel pour la production de données tomographiques (34), y compris une série de vues en coupe qui s'entrecroisent (40, 42, 44), chaque vue en coupe étant une image (54) contenant des points (52) avec des valeurs dérivées du moyennage d'une série de voxels (50) dans l'ensemble de données tomographiques, disposées transversalement à la section correspondante ; et une source thérapeutique (16) pouvant être contrôlée en réponse au feedback des données tomographiques pour la production d'un rayonnement thérapeutique.

2. Un appareil de radiothérapie conforme à la revendication 1 dans lequel les vues en coupe sont disposées dans un plan orthogonal.

3. Un appareil de radiothérapie conforme à la revendication 1 dans lequel on a préparé au minimum trois vues en coupe.

4. Un appareil de radiothérapie conforme à la revendication 3 dans lequel les vues se croisent en grande partie à l'isocentre de la source thérapeutique.

5. Un appareil de radiothérapie conforme à la revendication 1 dans lequel la série de voxels est disposée dans un plan orthogonal par rapport à la section correspondante.

6. Un appareil de radiothérapie conforme à la revendication 5 dans lequel la série de voxels est disposée dans un plan linéaire.

7. Un appareil de radiothérapie conforme à une quelconque des revendications 5 à 6 dans lequel on emploie de 5 à 20 voxels.

8. Un appareil de radiothérapie conforme à une quelconque des revendications 5 à 7 dans lequel on emploie de 7 à 15 voxels.

9. Un appareil de radiothérapie conforme à une quelconque des revendications 5 à 8 dans lequel on emploie environ 10 voxels.

10. Un appareil de radiothérapie conforme à une quelconque des revendications précédentes, comprenant un dispositif d'affichage pour l'affichage des vues en coupe.

11. Un appareil de radiothérapie conforme à la revendication 10 dans lequel la source thérapeutique est contrôlée en fonction d'instructions reçues d'un opérateur, à travers un dispositif d'entrée.

12. Un appareil de radiothérapie conforme à la revendication 11 dans lequel le dispositif d'entrée est en corrélation avec le dispositif d'affichage.

13. Un appareil de radiothérapie conforme à la revendication 12 dans lequel la corrélation s'effectue par le biais d'une image superposée sur le dispositif d'affichage, pouvant être déplacée en réponse à l'activation du dispositif d'entrée.

14. Un appareil de radiothérapie conforme à la revendication 13 dans lequel l'image superposée est dérivée d'un examen et d'un traitement précédents du patient.

15. Un appareil de radiothérapie conforme à la revendication 13 ou à la revendication 14, dans lequel l'image superposée est une esquisse.
